# EUROPEAN PATENT SPECIFICATION

(11) **EP 3 090 717 B1**
(45) Date of publication and mention of the grant of the patent: **27.12.2017**
(21) Application number: 15166852.2
(22) Date of filing: 07.05.2015
(51) Int. Cl.: A61G 1/044

(54) **BASKET STRETCHER**
KORBTRAGE
CHÂSSIS DE PANIER

(43) Date of publication of application: 09.11.2016
(73) Proprietor: IVECO MAGIRUS AG, 89079 Ulm (DE)
(72) Inventor: Huehn, Alexander, 89075 Ulm (DE)
(74) Representative: Franzolin, Luigi

(56) References cited:
- WO-A1-2012/142636
- US-A1- 2003 150 059
- US-A1- 2009 288 255
- US-A1- 2013 263 376
- US-B1- 6 363 936

## Description

The present invention refers to a basket stretcher, comprising the features of the preamble of claim 1.

Basket stretchers are used in rescue situations for the transport of patients from a dangerous area to a safe place. In a common embodiment such basket stretchers comprise a trough-shaped stretcher body for accommodating the patient. The outer rim of the stretcher body often comprises grip holes for lifting and carrying the basket stretcher. Moreover, a lifting gear is provided for attaching the basket stretcher to a lifting device, like the tip of a turnable ladder of a fire fighting vehicle. The lifting gear comprises a plurality of ropes that are fixed at different fixing points distributed along the rim of the stretcher body. In a common arrangement, four ropes are provided at four different fixing points that run together at a connection element to be fixed at the lifting device, such that the four ropes span a pyramidal space above the stretcher body.

The patient can be secured in a lying position within the stretcher body by means of a plurality of individual straps that are distributed along the rim of the stretcher body such that they extend across the open top side of the stretcher body. Each of these straps spans over one body portion of the patient, e. g. over the feet, the thighs, the waist, the patient's chest, etc. In any position of the basket stretcher, it must be prevented that the patient changes her/his position or even falls or slips out of the stretcher body.

The safety prescriptions for rescue devices require a back-up system for securing patients in transport devices. For this reason the patient lying within the basket stretcher must be secured by an additional safety gear. According to a common safety standard, such a safety gear must comprise a securing strap loop provided such that the person to be rescued is held by straps running from her/his back under the arms at the height of her/his chest. Such a back-up safety gear is an additional device to be handled in a rescue situation, making the preparation of the patient's transport more elaborate and time consuming. On the other hand, each additional safety device to be handled and secured imposes the danger of handling mistakes. Up to now there is no satisfying solution to integrate a back-up system according to the safety rules into a standard basket stretcher, as described above.

Moreover, there are often situations in which a patient must be lifted through a narrow shaft or well. In these situations the basket stretcher must be positioned vertically. In this position it must be avoided under all circumstances that the patient slips under the straps in the downward direction. The additional back-up safety gear mentioned above must also be present in this situation. However, it is often difficult to adapt the length of the conventional safety gear when the patient is prepared for transport within the basket stretcher resting on the ground, such that the length it still correct when the stretcher is lifted and tilts into the vertical orientaion when lifted.

It is therefore an object of the present invention to provide a basket stretcher of the above kind with a back-up safety gear for securing a patient in her/his lying position within the stretcher body that complies with the general safety prescriptions and does not impose much effort to the members of the rescue crew to apply the safety gear to the patient, also keeping the danger of handling mistakes low. Another object is to provide this basket stretcher such that a time consuming and error-prone adaption of the length of the safety gear is not necessary when the basket stretcher is prepared to be used in a vertical orientation.

These objects are achieved by a basket stretcher comprising the features of present main claim.

The basket stretcher according to the present invention comprises a safety harness that is located at one end portion of the stretcher body, where the head and chest portion of the patient can be arranged. This safety harness comprises a strap loop formed by one strap with connectable ends for surrounding the patient's chest. The safety harness further comprises at least two fixing straps for fixing the strap loop to opposite sides of the stretcher body. Each fixing strap connects the strap loop with one of two opposite fixing points of the lifting gear.

This safety harness can be positioned at the patient's body by laying the strap of the strap loop onto the bottom of the stretcher body, optionally guided through upholstery parts at the inner side of the stretcher body, such that the patient can be positioned on top of this strap with her/his back. Afterwards the ends of this strap can be guided laterally at the patient's body below her/his arm pits onto the chest to be connected on top of the chest. After connecting the ends of this strap to close the strap loop, the strap loop surrounds the upper body portion, i.e. the chest and back of the patient completely.

At the same time, the fixing straps connect the portion of the strap loop that is positioned on the patient's back with the fixing points of the lifting gear at the rim of the stretcher body, such that there is a direct connection between the safety harness and the lifting gear. A time consuming and error-prone adaption of the safety gear is not necessary even if the basket stretcher is to be used in a vertical orientation.

The safety harness according to the present invention has the function of fixing the patient within the stretcher body, and forming a back-up system according to the requirements of the prescriptions for rescue devices. In practice it is possible to replace one of the common individual straps for fixing the patient's chest within the stretcher body by a safety harness according to the present invention, having the same fixing function as the individual straps running in a traverse direction but surrounding the upper body portion of the patient completely to hold the weight of the patient even in cases in which a basket stretcher takes an almost vertical position. In case of breaking or malfunctioning of the basket stretcher or its parts, including the individual straps, the patient will still be held by the safety harness which is connected to the lifting gear directly and operates independent of other parts of the basket stretcher.

These and other objects and advantages of the present invention will be explained in more detail and elucidated with the help of the following drawings, which will be described hereinafter.
- Fig. 1: is a top view of an embodiment of a basket stretcher according to one embodiment of the present invention; and
- Fig. 2: is a perspective view of the basket stretcher of Fig. 1 in a tilted position.

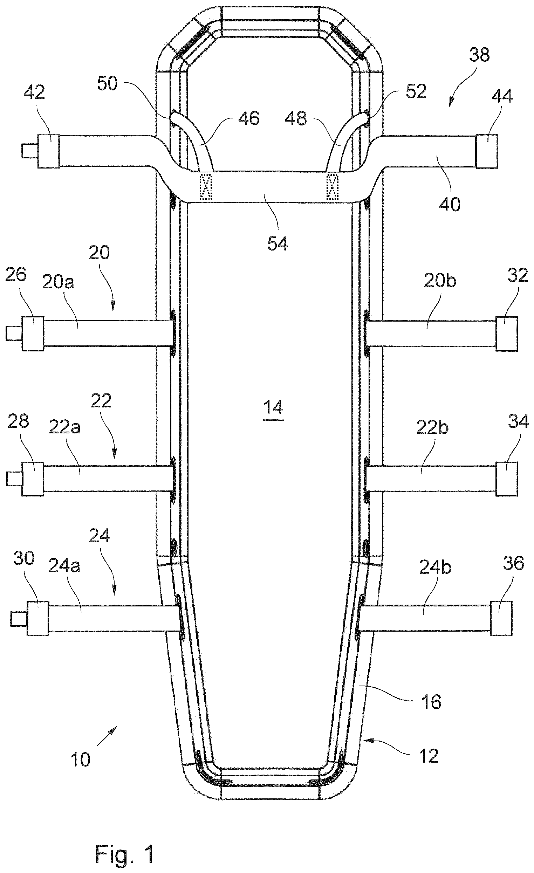

The basket stretcher 10 shown in Fig. 1 comprises a generally trough-shaped stretcher body 12 for accommodating a patient. In the position shown in Fig. 1, the top portion of the stretcher body 12 is provided for taking the upper body portion of the patient and represents a head portion, while the lower portion in the figure represent the foot end of the basket stretcher 10.

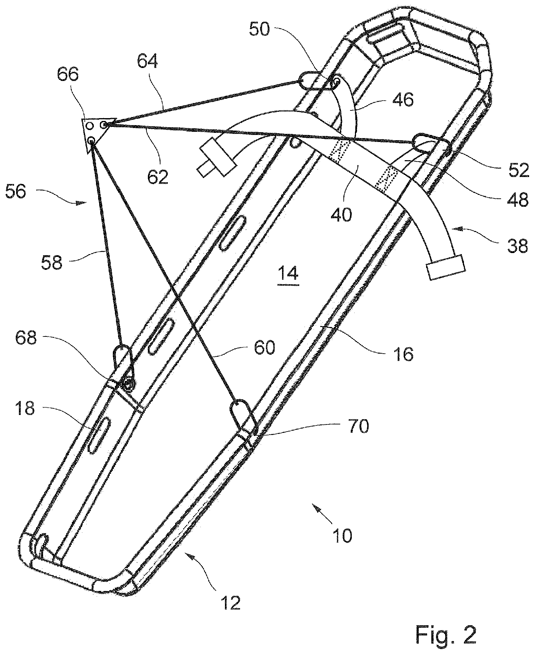

The stretcher body 12 comprises a flat bottom 14 and a raised outer rim 16 that is provided with grip holes 18 (Fig. 2). Three individual straps 20, 22, 24 are distributed along the rim 16 of the stretcher body 12 such that these straps 20, 22, 24 can extend across the opened top side of the stretcher body 12 in a traverse direction and parallel to each other. In other embodiments not shown here, the individual straps may cross each other. Each of these straps 20, 22, 24 comprises two strap sections 20a, 20b; 22a, 22b; and 24a, 24b, with one end being fixed to the rim 16 of the stretcher body 12 and its free end being equipped with a connector 26, 28, 30 to be connected with a respective complementary connector 32, 34, 36 of the respective other section of this strap. For example, the uppermost strap 20 in Fig. 1 for extending across an upper body portion of the patient comprises two strap sections 20a, 20b, each of these strap sections 20a, 20b extending from opposite sides of the rim 16, with a left strap section 20a being equipped at its free end with a connector 26 to be connected with a connector 32 of the right strap section 20b. The other straps 22, 24 comprise strap sections to be connected in the same way, to extend across the thighs and the legs of the patient individually, and so their individual function shall not be described here further for reasons of brevity.

At the head portion of the basket stretcher 10, a safety harness 38 is provided to secure the uppermost portion of the patient's body. This safety harness 38 comprises one strap 40 extending across the bottom 14 of the stretcher body 12 and over its rim 16, with its free ends being equipped with complementary connectors 42, 44 to be connected with each other. In the connected state of the connectors 42, 44, the strap 40 forms one single strap loop for surrounding the patient's chest. The connectors 42, 44 may be provided such that they do not fit to the connectors 26, 28, 30, 32, 34, 36 of the individual straps 20, 22, 24, to avoid wrong connections between the strap 40 and the other strap sections.

The safety harness 38 further comprises two fixing straps 46, 48 connecting the strap 40 of the strap loop with the rim 16 of the stretcher body 12 at opposite lateral sides. Namely, a left fixing strap 46 connects a left portion in Fig. 1 of the strap 40 with a left fixing point 50 on the left side in Fig. 1 at the rim 16, while an opposite right fixing strap 48 connects the strap 40 with a right fixing point 52 opposite to the left fixing point 50. By this arrangement, the strap 40 is connected to opposite left and right sides of the rim 16 by means of the two fixing straps 46, 48. The fixing straps 46, 48 are connected to the strap 40 at positions spaced apart from each other by a distance corresponding almost to the width of the bottom 14 of the stretcher body 12 in a traverse direction.

A patient is secured within the basket stretcher 10 as follows. First, the individual straps 20, 22, 24 as well as the strap 40 of the safety harness 38 forming the strap loop are positioned in the way as demonstrated in Fig. 1, i. e. the different sections 20a, 20b, 22a, 22b, 24a, 24b of the individual straps 20, 22, 24 are positioned out of the stretcher body 12, while the central portion 54 of the strap 40 of the safety harness 38 lies flat on the bottom 14 of the stretcher body 12, with the connectors 42, 44 at its free ends also positioned outside the stretcher body 12.

In this position the patient is positioned lying within the stretcher body 12, so that its back rests on the central portion 54 of the strap 40.

Afterwards the free ends of the strap 40 are guided under the arm pits of the patient to the top of her/his chest, and the connectors 42,44 are closed to close the loop of the strap 40. The lower portions of the patient's body are secured by closing the connectors 26, 32, 28, 34 and 30, 36 of the individual other straps 20, 22, 24, so that these straps 20, 22, 24 extend across other portions of the patient's body.

As can be taken from Fig. 2, the fixing points 50, 52 for the fixing straps 46, 48 of the safety harness 38 also serve as fixing points for a lifting gear 56 for attaching the basket stretcher 10 to a lifting device, like, for example, a tip of a turnable ladder. The lifting gear 56 comprises four ropes 58, 60, 62, 64 running from one common connection element 66 towards four distributed fixing points 50, 52, 68, 70 at the rim 16 of the stretcher body 12. The upper two fixing points 50, 52 are the same as the fixing points for the fixing straps 46, 48, as described above, such that the safety harness 38 is directly connected to the lifting gear 56. The lower two fixing points 68, 70 are located in a longitudinal distance from these upper fixing points 50, 52, respectively. With the ropes 58, 60, 62, 64 of the lifting gear 56 being connected with each one end to the fixing points 50, 52, 68, 70 and their other ends being connected to the connection element 66, these ropes 58, 60, 62, 64 span a pyramidal space above the basket stretcher 10.

In Fig. 2, the safety harness 38 is shown again in an open state, while the individual straps 20, 22, 24 are omitted for simplicity. In the tilted position shown in Fig. 2, a patient's body can still be held by the safety harness 38 in the closed state of the connectors 42, 44 of the strap loop. That is, it is prevented that the patient can slip on the bottom 14 of the stretcher body 12 below the individual straps 22, 24. The safety harness 38 has not only a function of fixing the patient within the basket stretcher 10 but also to provide a back-up system being connected to the lifting gear 56 directly for securing the patient.

## Claims

1. Basket stretcher (10), comprising a trough-shaped stretcher body (12) for accommodating a patient, a lifting gear (56) with a plurality of ropes (58, 60, 62, 64) to be fixed at different fixing points (50, 52, 68, 70) distributed along an upper rim (16) of the stretcher body (12), and a plurality of individual straps (20, 22, 24) distributed along the upper rim (16) of the stretcher body (12) to extend across the open top side of the stretcher body (12), **characterized by** a safety harness (38) located at one end portion of the stretcher body (12) comprising a strap loop formed by one strap (40) with connectable ends (42, 44) for surrounding the patient's chest and at least two fixing straps (46, 48) for fixing the strap loop to opposite sides of the stretcher body (12), each fixing strap (46, 48) connecting a connection point at the strap loop with one of two opposite fixing points (50, 52) of the lifting gear (56).

## Patentansprüche

1. Korbtrage (10), umfassend einen trogförmigen Tragenkörper (12) zur Aufnahme eines Patienten, ein Hubgeschirr (56) mit einer Mehrzahl von Seilen (58,60,62,64) zur Befestigung an unterschiedlichen Befestigungspunkten (50,52,68,70), die entlang eines oberen Randes (16) des Tragenkörpers (12) verteilt sind, und einer Mehrzahl einzelner Gurte (20,22,24), die entlang des oberen Randes (16) des Tragenkörpers (12) verteilt sind, derart, dass sie sich über die offene Oberseite des Tragenkörpers (12) erstrecken, **gekennzeichnet durch** ein Sicherungsgurtgeschirr (38), das an einem Endbereich des Tragenkörpers (12) angeordnet ist und eine Gurtschlaufe umfasst, die durch einen Gurt (40) mit miteinander verbindbaren Enden (42,44) zur Einfassung des Brustkorbs des Patienten, und durch zwei Befestigungsgurte (46,48) zur Befestigung der Gurtschlaufe an gegenüberliegenden Seiten des Tragenkörpers (12) gebildet wird, wobei jeder Befestigungsgurt (46,48) am Verbindungspunkt an der Gurtschlaufe mit einem von zwei gegenüberliegenden Befestigungspunkten (50,52) des Hubgeschirrs (56) verbindet.

## Revendications

1. Civière (10) comprenant un corps de civière en forme de barquette (12) pour recevoir un patient, un mécanisme de levage (56) muni d'une pluralité de cordes (58, 60, 62, 64) destinées à être fixées au niveau de différents points de fixation (50, 52, 68, 70) qui sont distribués le long d'un rebord supérieur (16) du corps de civière (12), et une pluralité de sangles individuelles (20, 22, 24) qui sont distribuées le long du rebord supérieur (16) du corps de civière (12) de telle sorte qu'elles s'étendent sur le côté supérieur ouvert du corps de civière (12), **caractérisée par** un harnais de sécurité (38) qui est situé au niveau d'une partie d'extrémité du corps de civière (12) comprenant une boucle de sangles qui est formée par une sangle (40) munie d'extrémités susceptibles d'être connectées (42, 44) pour entourer la poitrine d'un patient et par au moins deux sangles de fixation (46, 48) pour fixer la boucle de sangles à des côtés opposés du corps de civière (12), chaque sangle de fixation (46, 48) connectant un point de connexion au niveau de la boucle de sangles à l'un de deux points de fixation opposés (50, 52) du mécanisme de levage (56).
